# EUROPEAN PATENT APPLICATION

(11) **EP 1 666 076 A2**
(43) Date of publication of application: **07.06.2006**
(21) Application number: 05024220.5
(22) Date of filing: 07.11.2005
(51) Int. Cl.: A61L 27/04, A61L 27/30, A61L 27/32, A61L 27/38

(54) **Material for repairing biological tissues and method of manufacturing the same**

(30) Priority: 15.11.2004 JP 2004330417; 25.04.2005 JP 2005126591
(71) Applicant: Olympus Corporation, Tokyo 151-0072 (JP); Tsuchiya, Toshie, Setagaya-ku Tokyo 158-0094 (JP); Isama, Kazuo, Tokyo 113-0001 (JP); Tamai, Masato, Yokohama-shi Kanagawa 227-0044 (JP)
(72) Inventor: Tamura, Tomoaki, Hino-shi Tokyo 191-0032 (JP); Tsuchiya, Toshie, Tokyo 158-0094 (JP); Isama, Kazuo, Tokyo 113-0001 (JP); Tamai, Masato, Yokohama-shi Kanagawa 227-0044 (JP); Hakamatsuka, Yasuharu, Akishima-shi Tokyo 196-0033 (JP)
(74) Representative: von Hellfeld, Axel

(57) **Abstract**

Osteoblast activity is promoted and a large amount of extracellular substrates are produced so as to promote cell proliferation and/or differentiation. A material for repairing biological tissues, the material comprising a biocompatible material containing at least one of niobium and tantalum, is provided. These metal salts are not toxic even at high concentrations, and can activate osteoblasts to increase the production quantity of extracellular substrates. The material for repairing biological tissues is manufactured by mixing a metal salt solution, a calcium compound, and a phosphate compound (S4), then coprecipitating the metal-containing calcium phosphate.

## Description

### BACKGROUND OF THE INVENTION

### 1. FIELD OF THE INVENTION

The present invention relates to a material for repairing biological tissues, a product for repairing biological tissues, and a method of manufacturing the material for repairing biological tissues.

This application is based on Japanese Patent Applications Nos. 2004-330417 and 2005-126591, the contents of which are incorporated herein by reference.

### 2. DESCRIPTION OF RELATED ART

Recently, it has become possible to repair a defect part of bone caused by osteoncus extraction, trauma, or the like, by regenerating the bone by filling a bone substitute. For such a bone substitute, hydroxyapatite (HAP) and tricalcium phosphate (TCP) are known. However, from the viewpoint of leaving no foreign matter inside the body, a scaffold made of a porous calcium phosphate material, for example, such as β-TCP is used. If the β-TCP is left in contact with bone cells of a defect part of bone, so-called remodeling is performed in which osteoclasts absorb the β-TCP, and osteoblasts form a new bone. That is, the bone substitute filled in the defect part of the bone is replaced by autologous bone as time goes by (for example, refer to: Uemura and two others, "Tissue engineering in bone using biodegradable porous β-TCP material - OSferion, a new material strengthened in vivo", Medical Asahi, The Asahi Shimbun Company, October 1, 2001, Vol. 30, No. 10, p. 46-49 (hereunder, "Document 1")).

On the other hand, in order to improve the initial adhesiveness of cells and tissues onto a porous calcium phosphate material, there is known a technique wherein polymers such as collagens are placed onto the surface of the porous calcium phosphate material. As a binding material which stably binds polymers such as collagens onto the surface of the porous calcium phosphate material, there is proposed a material which is chemically modified with metal ions such as zinc. (For example, refer to Japanese Unexamined Patent Application, First Publication No. 2001-198208 ([0018] and the like) (hereunder, "Document 2"))

Moreover, metal biomedical materials such as stainless steel, cobalt/chromium alloy, titanium alloy, and the like are used for an medical implant device such as an artificial bone, an artificial joint, and a bone fixing material (for example, refer to: Kazuo Isama and Toshie Tsuchiya, "Effect of metal salts on the proliferation and differentiation of normal human osteoblasts", 23rd Annual Meeting of The Japanese Society for Biomaterials proceedings, p190 (hereunder, Document 3)).

As shown in Document 2 and Document 3, there exists a material for repairing biological tissues, and an medical implant device containing an abundance of metal ions such as zinc.

However, an abundance of metal ions such as zinc shown in Document 2 are toxic, and the higher concentration thereof causes concern of being a factor of inhibiting cell proliferation. Therefore, it is assumed that such metal ions are used within a range of very low concentration so as not to increase the toxicity.

Moreover, in Document 3, there are disclosed metal salts that are less toxic without inhibiting cell proliferation. However, throughout there is no more than a disclosure of metal medical implant devices such as an artificial bone, an artificial joint, a bone fixing material, and the like that do not inhibit cell proliferation, and there is no mention of any material for repairing biological tissues that positively promotes cell proliferation so as to regenerate biological tissues.

### BRIEF SUMMARY OF THE INVENTION

An object of the present invention is to provide a material for repairing biological tissues and a product for repairing biological tissues, which can promote the osteoblast activity and produce a large amount of extracellular substrates so as to promote cell proliferation and/or differentiation.

Furthermore, an object of the present invention is to provide a method of manufacturing a material for repairing biological tissues for readily manufacturing a material for repairing biological tissues, which can promote the osteoblast activity and produce a large amount of extracellular substrates so as to promote cell proliferation and/or differentiation.

In order to achieve the above objects, the present invention provides a material for repairing biological tissues, the material comprising a biocompatible material containing at least one of niobium and tantalum.

The present inventors found that both of niobium and tantalum do not inhibit the cell proliferation, but rather promote the osteoblast activity, functioning to increase the production quantity of extracellular substrate.

Here, according to the material for repairing biological tissues of the present invention which utilizes this finding, by filling this material for repairing biological tissues into a defect part of biological tissue, the surrounding osteoblasts are activated to produce an abundance of extracellular substrates so that the defect part can be rapidly repaired.

Moreover, by having the material for repairing biological tissues of the invention contain at least one of niobium and tantalum in a porous calcium phosphate material, this is particularly filled in a defect part of bone so that the bone tissues can be rapidly repaired.

Moreover, the material for repairing biological tissues of the invention may be a bioglass having the surface coated with at least one of niobium and tantalum. Alternatively, the material for repairing biological tissues of the invention may be a biopolymer containing at least one of a metal salt of niobium and a metal salt of tantalum.

Furthermore, in the material for repairing biological tissues of the invention, it is effective if the total concentration of niobium and tantalum is more than 10⁻⁵ mol/l but not greater than 10⁻³ mol/l. In this case, the production quantity of extracellular substrate can be greatly increased by the differentiation of osteoblasts, and consequently a defect part of biological tissue can be effectively repaired.

The present invention provides a product for repairing biological tissues having cells seeded in the material for repairing biological tissues.

According to the product for repairing biological tissues of the invention, the seeded cells are activated so that the cells can be in a sufficiently activated state prior to being filled into a living body. That is, during the process where the material for repairing biological tissues is being absorbed into the cells, niobium or tantalum may be taken into the cells, causing a stronger effect. Consequently, it becomes possible to rapidly regenerate biological tissues after an operation.

Furthermore, the present invention provides a method of manufacturing a material for repairing biological tissues comprising mixing a metal salt solution, a calcium compound, and a phosphate compound so as to coprecipitate a metal together with calcium phosphate.

According to the method of manufacturing a material for repairing biological tissues of the present invention, since metals contained in the metal salt solution are coprecipitated when the calcium phosphate is being precipitated, a material for repairing biological tissues, the material comprising a metal-containing biocompatible calcium phosphate, can be readily manufactured.

In the method of manufacturing a material for repairing biological tissues of the invention, a mixture of a metal salt solution and a phosphate compound may be mixed with a calcium compound so as to coprecipitate a metal together with calcium phosphate.

By so doing, a mixture of a metal salt solution and a phosphate compound can be made homogenously at a constant concentration prior to generating the precipitation. Consequently, metals can be distributed homogenously at a constant concentration in the obtained metal-containing calcium phosphate.

In the method of manufacturing a material for repairing biological tissues of the invention, the metal salt solution is preferably a mixed solution of α-hydroxyketone and alkanolamine, and more preferably a mixed solution of hydroxyacetone and aminoethanol, having a metal salt dissolved therein.

Moreover, in the method of manufacturing a material for repairing biological tissues of the invention, a metal constituting the metal salt is preferably at least either one of niobium and tantalum. Furthermore, the total content of niobium and tantalum is preferably 1 to 10 atom % with respect to the calcium content.

As described above, it is found that both of niobium and tantalum do not inhibit the cell proliferation, but rather promote the osteoblast activity, functioning to increase the production quantity of extracellular substrate. According to the method of manufacturing a material for repairing biological tissues of the present invention, by filling this material for repairing biological tissues into a defect part of biological tissue, the surrounding osteoblasts are activated to produce an abundance of extracellular substrates. Therefore the material for repairing biological tissues that can rapidly repair the defect part can be readily manufactured.

The material for repairing biological tissues and the product for repairing biological tissues of the present invention demonstrate an effect of activating the osteoblasts to increase the production quantity of extracellular substrates, so that the filled biological tissue can be rapidly repaired.

Consequently, by using the material for repairing biological tissues of the present invention as a base material for culturing osteoblasts, osteoblasts can be cultured in a highly active state. Moreover, by using this as a base material for obtaining a cultured bone, a cultured bone having a high osteoblast activity can be obtained, and a cultured bone having abundant extracellular substrates can be obtained. Furthermore, by using this as a bone substitute, osteoblasts can be activated so as to rapidly repair the defect part and the like. By using this as a therapeutic drug for osteoporosis, osteoblasts can be activated so as to enable curing of osteoporosis by the recalcifying effect of the extracellular substrate. Moreover, by coating this on the surface of a metal such as of an artificial joint, without causing osteolysis of the grafted part due to the abrasion between itself and the bone, bone recalcification in the abrasion part can be expected. Therefore, an expected effect is that the period of time for exchanging the artificial joint or for readjusting the coating material due to the osteolysis can be extended or becomes unnecessary.

The method of manufacturing a material for repairing biological tissues of the present invention demonstrates an effect of readily manufacturing a material for repairing biological tissues, the material comprising a metal-containing calcium phosphate, which activates the osteoblasts to increase the production quantity of extracellular substrates so that the filled biological tissue can be rapidly repaired.

### BRIEF DESCRIPTION OF THE SEVERAL VIEWS OF THE DRAWINGS

FIG. 1 is a graph showing the relation between the concentration of metal salts contained in a material for repairing biological tissues according to a first embodiment of the present invention, and the proliferation rate of osteoblasts.
FIG. 2 is a graph showing the relation between the concentration of metal salts contained in the material for repairing biological tissues according to the first embodiment of the present invention, and the differentiation rate of osteoblasts.
FIG. 3 is a flowchart showing a method of manufacturing a material for repairing biological tissues according to a second embodiment of the present invention.
FIG. 4 is a flowchart showing a method of adjusting niobium pentachloride solution in the manufacturing method of FIG. 3.
FIG. 5 is a graph showing the relation between the niobium concentration in the niobium pentachloride-containing hydroxyapatite manufactured by the manufacturing method of FIG. 3, and the alkaline phosphatase activity.
FIG. 6 is a graph showing the relation between the niobium concentration in the niobium pentachloride-containing β-TCP manufactured by the manufacturing method of FIG. 3, and the alkaline phosphatase activity.

### DETAILED DESCRIPTION OF THE INVENTION

### First Embodiment

Hereunder is a description of a material for repairing biological tissues and a product for repairing biological tissues according to a first embodiment of the present invention.

The material for repairing biological tissues according to the present embodiment comprises, for example, a porous calcium phosphate material such as porous β-TCP material containing at least either one of a metal salt of niobium (for example, niobium pentachloride - NbCl₅) and a metal salt of tantalum (for example, tantalum pentachloride : TaCl₅).

The concentration of such metal ions is more than 10⁻⁵ mol/l but not greater than 10⁻³ mol/l.

By filling the material for repairing biological tissues according to the present embodiment into a defect part of bone that has been formed for example by curetting a tumor formed in a bone tissue, the metal salts of niobium or tantalum contained therein become niobium ions or tantalum ions to act on the surrounding osteoblasts. The osteoblast are activated by the effect of such metal ions, producing a large amount of extracellular substrates. As a result, the produced extracellular substrates further promote the growth of the osteoblasts, repeating the differentiation and proliferation, by which bone tissues are formed rapidly.

The action and effect in the present embodiment is based on an evaluation test below performed by the inventors.

The precondition of the evaluation test is as follows.

The material for repairing biological tissues of the present example was made as follows.
1. Firstly, calcium phosphate was synthesized by a coprecipitation method so that the Ca/P ratio was within a range of chemical composition between 1.50 and 1.68. Then, it was sintered at a temperature of 750°C or more to obtain calcium phosphate powder.
2. Next, the calcium phosphate powder made as above was added with water, surfactant, and Ta(OC₂H₅)₅ or Nb(OC₂H₅)₅, then stirred and mixed. The resulting foam material was then poured into a paper tray and dried at 80°C for 24 hours.
3. Then, the temperature was increased at the rate of 100°C per hour, and the material was sintered at a temperature of 900 to 1000°C for 30 minutes. As a result, niobium- or tantalum-containing porous calcium phosphate material was obtained.

Moreover, as a metal salt, in addition to niobium pentachloride (NbCl₅) or tantalum pentachloride (TaCl₅), the following were prepared as comparative examples: aluminum trichloride (AlCl₃), cadmium dichloride (CdCl₂), cobalt dichloride (CoCl₂), chromium trichloride (CrCl₃), copper dichloride (CuCl₂), iron dichloride (FeCl₂), iron trichloride (FeCl₃), manganese dichloride (MnCl₂), molybdenum pentachloride (MoCl₅), nickel dichloride (NiCl₂), titanium tetrachloride (TiCl₄), vanadium trichloride (VCl₃), zinc dichloride (ZnCl₂), and zirconium tetrachloride (ZrCl₄).

As a cell, normal human osteoblast NHOst (CC-2538, Clonetics) was used.

As a medium, α-MEM medium containing β-sodium glycerophosphate and fetal calf serum was used.

The cell culturing method was such that about 1×10⁴ osteoblasts were seeded in each well in a collagen type I coated 24 well microplate. Then after 24 hours the product was replaced with 1 ml of medium containing a prescribed concentration of metal salts, after which it was further replaced with a medium containing the same concentration of metal salts three times per week, and the culturing was performed for 2 weeks.

The proliferation rate and the differentiation rate of the cells cultured in the above manner were measured as follows.

The proliferation rate was measured as follows. 1 ml of medium containing 20 µl/ml of living cell measuring reagent, TetraColor ONE (Seikagaku Corporation) was added into each well. After 2 hours of culturing, the absorbancy at 450nm (control wavelength was 600nm) was measured. The proliferation rate of the respective experimental groups was obtained assuming that the absorbancy of the control group was 100 %.

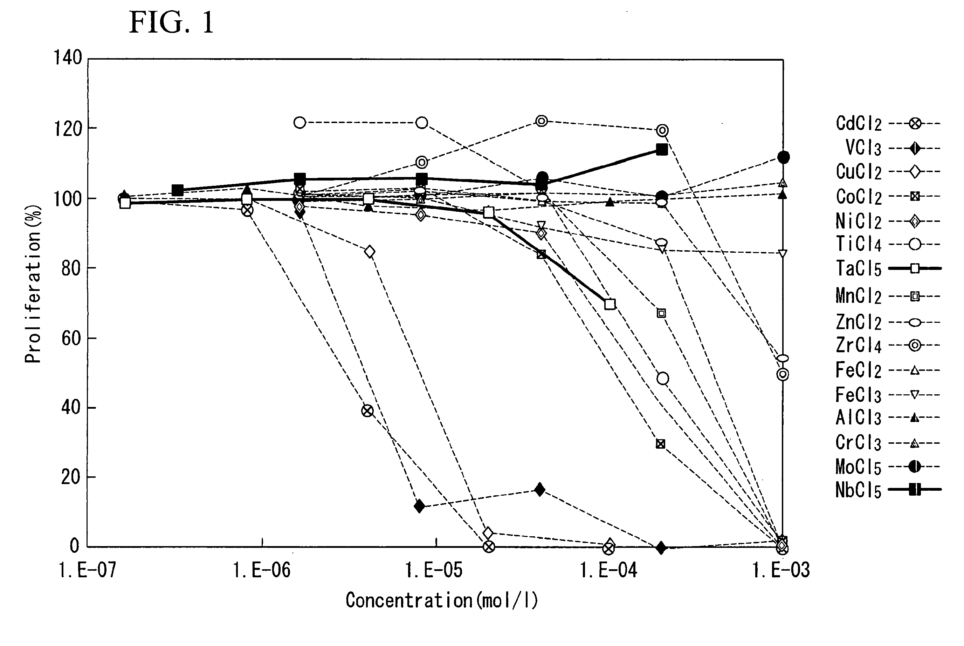

The measurement results are shown in FIG. 1. According to these, it was found that tantalum pentachloride (TaCl₅) only slightly decreased the proliferation rate of osteoblasts even at a concentration as high as 10⁻⁵mol/l or more, and niobium pentachloride (NbCl₅) increased the proliferation rate as its concentration became higher, while the other metal salts rapidly decreased the proliferation rate as their concentration became higher.

Since the metal salts other than niobium pentachloride (NbCl₅) and tantalum pentachloride (TaCl₅) are highly toxic, they decrease the proliferation rate of osteoblasts as their concentration become higher. However, since niobium pentachloride (NbCl₅) and tantalum pentachloride (TaCl₅) are less toxic or not toxic at all, they do not decrease the proliferation rate very much even if their concentration become higher, and further they function so as to increase the proliferation rate.

The differentiation rate was measured as follows. 1 ml of 0.1N hydrochloric acid was added into each well and left at room temperature for 15 hours to obtain the hydrochloric acid extract of each well. Using calcium C-Test Wako (Wako Pure Chemical Industries, Ltd.), 10 µl of hydrochloric acid extract was added with 1 ml of 0.88 M monoethanolamine buffer (pH 11.0), then 100 µl of 0.63 mM ortho-cresolphthalein complexone and 100 µl of 69 mM 8-quinolinol. After 15 minutes of reaction at room temperature, the absorbancy at 570 nm was measured to determine the calcium content per well. The differentiation rate of the respective experimental groups was obtained assuming that the calcium content of the control group was 100 %.

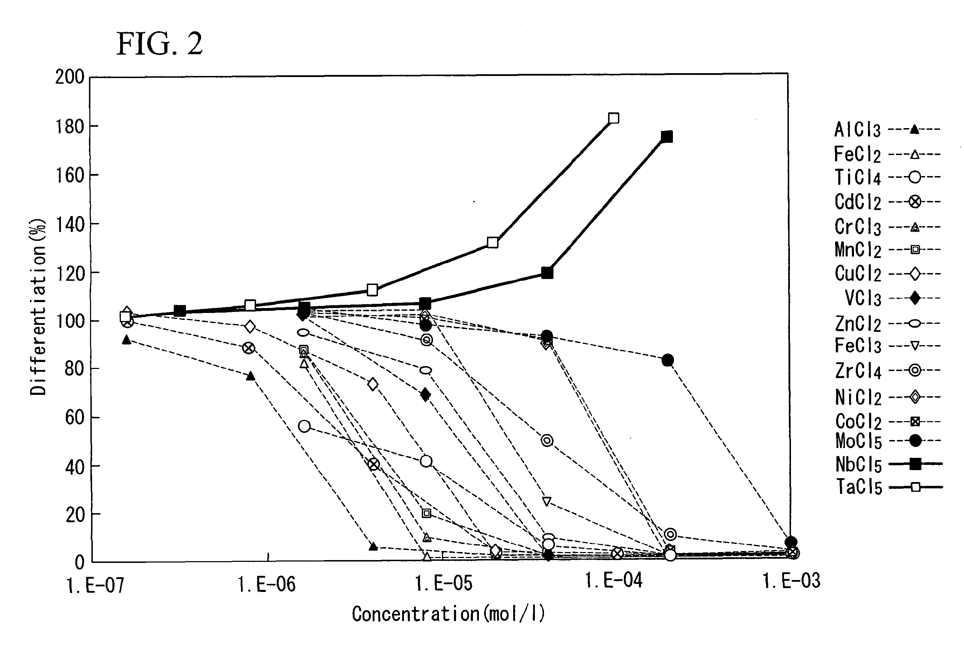

The measurement results are shown in FIG. 2. According to these, it was found that tantalum pentachloride (TaCl₅) and niobium pentachloride (NbCl₅) increased the differentiation rate as their concentration became higher, while the other metal salts rapidly decreased the differentiation rate as their concentration became higher.

As the differentiation rate becomes higher, the osteoblast is more activated to produce an abundance of extracellular substrates.

In this manner, the material for repairing biological tissues according to the present embodiment has an effect such that: by simply filling it into a defect part of biological tissue, the activity of the surrounding osteoblasts is remarkably increased so that the biological tissue can be rapidly repaired.

Consequently, by using this as a base material for culturing osteoblasts, osteoblasts can be cultured in a highly active state. Furthermore, by using this as a material for repairing bone, osteoblasts can be activated so as to rapidly repair the defect part and the like. By using this as a therapeutic drug for osteoporosis, osteoblasts can be activated so as to enable curing of osteoporosis by the recalcifying effect of the extracellular substrate. Moreover, by coating this on the surface of a metal such as of an artificial joint, without causing osteolysis of the grafted part due to the abrasion between itself and the bone, bone recalcification in the abrasion part can be expected. Therefore, an expected effect is that the period of time for exchanging the artificial joint or for readjusting the coating material due to the osteolysis can be extended or becomes unnecessary. Furthermore, it is also effective to apply to biological tissues other than bone tissues.

Moreover, by seeding and culturing mesenchymal stem cells using the material for repairing biological tissues according to the present embodiment as a base material, a product for repairing biological tissues having a high osteoblast activity can be obtained. Furthermore, according to such a product for repairing biological tissues, since extracellular substrates are abundantly contained, a defect part of biological tissue can be further rapidly repaired after grafting.

As an example of the base material of the material for repairing biological tissues, porous β-TCP material was used. However, instead of this, other calcium phosphate or any material may be used provided it has an affinity with the biological tissue, and more preferably it has a bioabsorbency. In particular, biocompatible porous ceramics, collagen, polylactic acid, polyglycolic acid, hyaluronic acid or combinations thereof may be used.

Moreover, in addition to the case where the base material of the material for repairing biological tissues contains niobium or tantalum, an other alloy material that is not positively added with niobium or tantalum can also be expected to effectively form a bone after being grafted into a living body. Considered examples as such an alloy material include conventional titanium alloy, stainless steel, cobalt/chromium alloy, and the like, which may be added with a niobium salt or a tantalum salt when the alloy is being produced. The effective concentration of niobium or tantalum is for example more than 10⁻⁵ mol/1 but not greater than 10⁻³ mol/1.

### Second Embodiment

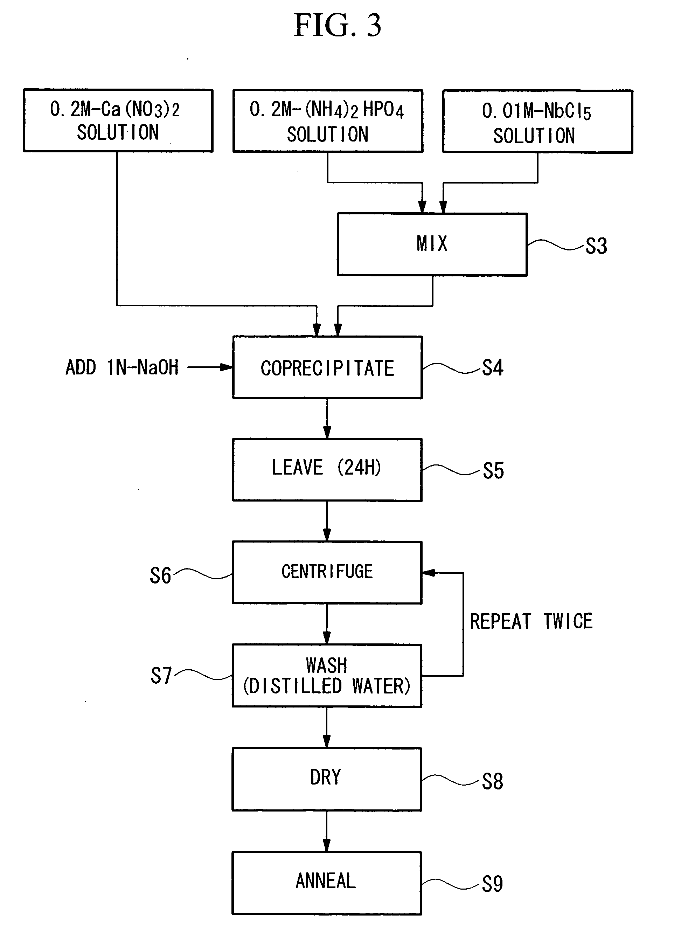

Hereunder is a description of a method of manufacturing a material for repairing biological tissues according to a second embodiment of the present invention, with reference to FIG. 3 to FIG. 6.

The method of manufacturing a material for repairing biological tissues according to the present embodiment is for manufacturing a material for repairing biological tissues which is for example, a porous calcium phosphate material such as porous β-TCP material containing at least either one of a metal salt of niobium (for example, niobium pentachloride : NbCl₅) and a metal salt of tantalum (for example, tantalum pentachloride : TaCl₅).

As shown in FIG. 3, in the manufacturing method according to the present embodiment, for example 0.2 M calcium nitrate solution was prepared as a calcium compound solution, and 0.2 M ammonium phosphate solution was prepared as a phosphate compound solution. Moreover, for example 0.01 M niobium pentachloride solution was prepared as a metal salt solution.

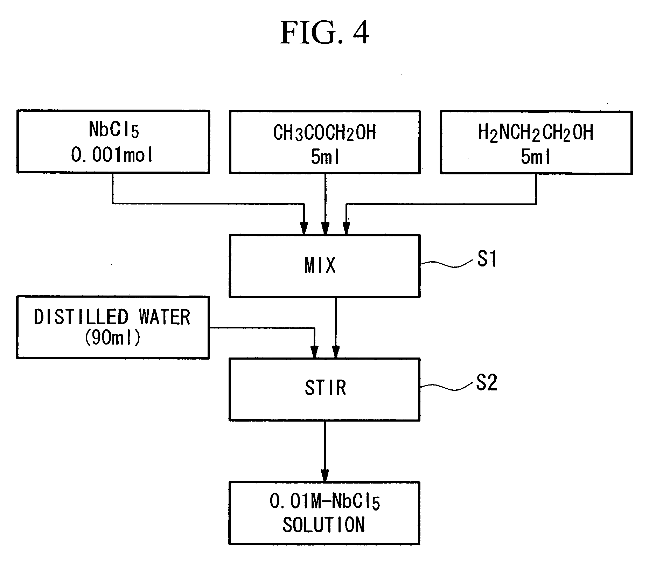

As shown in FIG. 4, 0.01 M niobium pentachloride solution was made by mixing 0.001 mol of niobium pentachloride, 5 ml of hydroxyacetone, and 5 ml of aminoethanol (S1), and adding 90 ml of distilled water, and stirring for 24 hours (S2).

Next, 0.2 M ammonium phosphate solution and 0.01 M niobium pentachloride solution were mixed (S3), and the mixture was mixed with 0.2 M calcium nitrate solution (S4). At this time, while the temperature was kept at room temperature, 1 basic sodium hydroxide was added so as to keep the solution at pH 10. Then, the solution was left in this condition for 24 hours (S5), enabling coprecipitation of niobium in the calcium phosphate that was being precipitated, so that a calcium deficient hydroxyapatite containing niobium with the ratio of calcium Ca and phosphorus P: Ca/P = 1.50 could be manufactured. By sintering this, β-TCP containing niobium could be manufactured.

Moreover, by adjusting the concentration or the amount of calcium compound solution or phosphate compound solution to be used, or by adjusting the temperature of the solution, a hydroxyapatite containing niobium with the ratio of calcium Ca and phosphorus P: Ca/P = 1.67 could be manufactured.

The calcium phosphate containing precipitated niobium was centrifuged at 3000 rpm for 2 minutes (S6), and washed with distilled water (S7). The procedure was repeated twice, and the product then dried at 50°C (S8). The temperature was increased at the rate of 5°C per minute between 500 and 800°C, and the product was left at 800°C for 2 hours, after which it was subjected slow cooling annealing (S9). The above procedure provides a powdery niobium-containing hydroxyapatite, a niobium-containing β-TCP, or composite porous calcium phosphate material comprising niobium-containing hydroxy-apatite/β-TCP.

The material for repairing biological tissues manufactured by the manufacturing method according to the present embodiment has a similar effect to that of the material for repairing biological tissues according to the first embodiment.

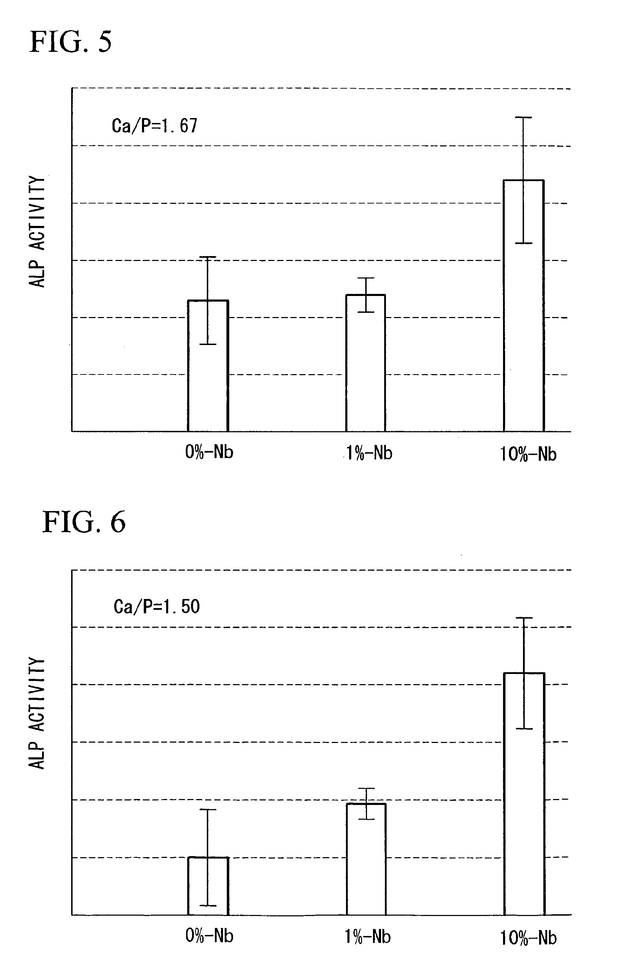

Hereunder is a description of the relation between the niobium content contained in calcium phosphate powder and the osteoblast activity, with reference to FIG. 5 and FIG. 6.

As the material for repairing biological tissues manufactured by the manufacturing method according to the present embodiment, calcium phosphate powder containing 1 atom % to 10 atom % of niobium with respect to calcium was prepared. As calcium phosphate, hydroxyapatite and β-TCP were prepared. Moreover, as comparative examples, calcium phosphate powder (hydroxyapatite and β-TCP) not containing niobium (0 atom %) that was manufactured without mixing with niobium pentachloride solution in the above manufacturing method, was prepared.

Then, osteoblasts were seeded over the calcium phosphate powder and cultured, after which the amount of alkaline phosphatase (ALP) produced from the cells was measured.

Specifically, 5×10⁴ cells/ml /well of normal human osteoblasts were seeded in a 24 well dish, and cultured in a medium containing α-MEM (Eagle's Minimum Essential Medium), 10 % FCS (fetal calf serum), and 10 mM β-glycerophosphate for a week. Moreover, ALP was measured by removing the medium, then adding 1 ml of mixture of 4 mM p-nitrophenylphosphate, 10 mM magnesium chloride, 0.1 mM zinc chloride, and 0.1 M glycine buffer, and measuring the absorbancy at 450 nm.

As a result, as shown in FIG. 5, in the calcium phosphate powder composed of hydroxyapatite, significant ALP activity was confirmed in the case where 10 atom % of niobium was contained compared to the case where no niobium was contained. Moreover, as shown in FIG. 6, in the porous calcium phosphate material composed of glycerophosphate-TCP, significant ALP activity was confirmed in both cases where 1 atom % and 10 atom % of niobium were contained compared to the case where no niobium was contained.

In this manner, the method of manufacturing the material for repairing biological tissues according to the present embodiment has an advantage of being able to readily manufacture the material for repairing biological tissues which can activate the osteoblasts so as to rapidly repair a defect part of biological tissue.

In the present embodiment, as an example of the metal salt solution, niobium pentachloride solution was used. However, instead of this, tantalum pentachloride (TaCl₅) solution may be used.

Similar to niobium, tantalum is less toxic or not toxic at all, and it is expected that it does not decrease the proliferation rate very much even if its concentration becomes higher, and further it functions so as to increase the proliferation rate.

As an example of a calcium compound solution, a calcium nitrate solution was employed. However, instead of this, a calcium chloride solution may be used.

Moreover, as an example of a metal salt solution, a niobium pentachloride solution was used. However, instead of this, a tantalum pentachloride (TaCl₅) solution may be used.

Moreover, the bone substitute made of niobium-containing calcium phosphate powder was shown as an example. However, instead of this, the material for repairing biological tissues can be used as a base material for culturing osteoblasts. By using this as a therapeutic drug for osteoporosis, osteoblasts can be activated so as to enable curing of osteoporosis by the recalcifying effect of the extracellular substrate. Moreover, by coating this on the surface of a metal such as of an artificial joint, without causing osteolysis of the grafted part due to the abrasion between itself and the bone, bone recalcification in the abrasion part can be expected. Therefore, an expected effect is that the period of time for exchanging the artificial joint or for readjusting the coating material due to the osteolysis can be extended or becomes unnecessary. Furthermore, it is also effective to apply to biological tissues other than bone tissues.

Moreover, by seeding and culturing mesenchymal stem cells using the material for repairing biological tissues manufactured by the manufacturing method according to the present embodiment as a base material, a product for repairing biological tissues having a high osteoblast activity can be obtained. Furthermore, according to such a product for repairing biological tissues, since extracellular substrates are abundantly contained, a defect part of biological tissue can be further rapidly repaired after grafting.

## Claims

1. A material for repairing biological tissues, the material comprising a biocompatible material containing at least one of niobium and tantalum.

2. A material for repairing biological tissues according to claim 1, comprising a porous calcium phosphate material.

3. A material for repairing biological tissues according to claim 1, comprising a bioglass having the surface coated with at least one of niobium and tantalum.

4. A material for repairing biological tissues according to claim 1, comprising a biopolymer.

5. A material for repairing biological tissues according to claim 1, wherein a total concentration of niobium and tantalum is more than 10⁻⁵ mol/l but not greater than 10⁻³ mol/l.

6. A product for repairing biological tissues having cells seeded in the material for repairing biological tissues according to claim 1.

7. A method of manufacturing a material for repairing biological tissues comprising mixing a metal salt solution, a calcium compound, and a phosphate compound so as to coprecipitate a metal together with calcium phosphate.

8. A method of manufacturing a material for repairing biological tissues comprising mixing a mixture of a metal salt solution and a phosphate compound with a calcium compound so as to coprecipitate a metal together with calcium phosphate.

9. A method of manufacturing a material for repairing biological tissues according to claim 7, wherein said metal salt solution is a mixed solution of α-hydroxyketone and alkanolamine having a metal salt dissolved therein.

10. A method of manufacturing a material for repairing biological tissues according to claim 8, wherein said metal salt solution is a mixed solution of α-hydroxyketone and alkanolamine having a metal salt dissolved therein.

11. A method of manufacturing a material for repairing biological tissues according to claim 7, wherein a metal constituting said metal salt is at least either one of niobium and tantalum.

12. A method of manufacturing a material for repairing biological tissues according to claim 8, wherein a metal constituting said metal salt is at least either one of niobium and tantalum.

13. A method of manufacturing a material for repairing biological tissues according to claim 11, wherein a total content of niobium and tantalum is 1 to 10 atom % with respect to the calcium content.

14. A method of manufacturing a material for repairing biological tissues according to claim 12, wherein a total content of niobium and tantalum is 1 to 10 atom % with respect to the calcium content.
